# EUROPEAN PATENT APPLICATION

(11) **EP 2 713 165 A1**
(43) Date of publication of application: **02.04.2014**
(21) Application number: 12306180.6
(22) Date of filing: 28.09.2012
(51) Int. Cl.: G01N 33/68

(54) **Use of vanin-1 as a biomarker of inflammatory bowel diseases**

(71) Applicant: INSERM (Institut National de la Santé et de la Recherche Médicale), 75013 Paris (FR)
(72) Inventor: The designation of the inventor has not yet been filed
(74) Representative: Hirsch, Denise Marie

(57) **Abstract**

The present invention provides vanin-1 (VNN1) as a biomarker for inflammatory bowel diseases. More particularly, VNN1 may be suitable for the diagnosis of inflammatory bowel disease (IBD), for the differential diagnosis between ulcerative colitis and Crohn's Disease, for monitoring the progression of an IBD, for detecting a bowel epithelial lesion in IBD, for monitoring treatments of IBD.

## Description

### FIELD OF THE INVENTION:

The present invention relates to the use of vanin-1 as a biomarker of inflammatory bowel diseases.

### BACKGROUND OF THE INVENTION:

Inflammatory bowel diseases (IBD), such as Crohn's disease (CD) and ulcerative colitis (UC) are severe and relapsing immunologically mediated chronic disorders of the gastrointestinal tract. IBDs are heterogeneous diseases characterized by various genetic abnormalities that lead to overly aggressive inflammatory responses to a subset of commensal enteric bacteria. Crohn's disease can affect all parts of the digestive tract and specially the ileum and/or colon and leads to mucosal ulcerations, fistula, and deep infiltration of inflammatory cells in the bowel wall. Ulcerative colitis often involves the lower part of the colon and the rectum and mucosal inflammation may extend to the caecum in a contiguous pattern. In clinical practice, 20 to 30% of patients with IBD colitis cannot be classified as CD or UC based upon usual endoscopic, radiologic, and histopathologic criteria, though this distinction may be crucial to guide therapeutic choices, especially when colonic resection is discussed. Similarly, the sensitivity of serological markers (autoantibodies to neutrophils [ANCA, pANCA] and antimicrobial antibodies [ASCA, anti-OmpC, anti-I2, and anti-CBir1]) remains insufficient to discriminate between CD and UC. On the other hand, the aetiology of IBDs and the cause of flare still remain largely unknown and specific biomarkers of CD and UC are also needed to assess an early diagnosis. To date, strictly specific biomarkers remain difficult to elect.

Mouse Vanin (Vnn) genes code for the two pantetheinase isoforms Vnn1 and Vnn3 (Martin et al., 2001). Pantetheinase hydrolyzes pantetheine to pantothenic acid (vitamin B5) and the amino-thiol cysteamine (Dupre and Cavallini, 1979). Cysteamine is thought to act by engaging mixed disulfides with cysteines regulating the function of target enzymes (Chu et al., 2005; Di Leandro et al., 2008; Griffith et al., 1977; Lebo and Kredich, 1978; Martin et al., 2004). Although discovered a long time ago, the biological function of pantetheinase remains an enigma. The Vnn1 pantetheinase is expressed at the membrane of epithelial cells by a GPI anchor (Aurrand-Lions et al., 1996). Using Vnn1 deficient mice, Vnn1 was shown to regulate gut inflammation (Berruyer et al., 2006; Martin et al., 2004) and granuloma formation during bacterial infections (Meghari et al., 2007). In addition it exerts a cytoprotective effect on islet beta cells and its deficiency exacerbates the severity of type 1 diabetes in NOD mice (Roisin-Bouffay et al., 2008). The other isoform, Vnn3 has no membrane anchoring domain and could be secreted (Martin et al., 2001). It is preferentially expressed by hematopoietic cell subsets and contributes to the resistance to malaria infection (Min-Oo et al., 2007). Therefore, the amount of pantetheinase activity modulates the evolution of various pathologies. Vnn1 gene expression is developmentally regulated (Bowles et al., 2000; Grimmond et al., 2000; Johnson et al., 2008). In adult mouse, Vnn1 is strictly epithelial but in a given tissue, expression is quite heterogeneous (Pitari et al., 2000) and can be further modulated by oxidative stress (Berruyer et al., 2004). There are several indications that Vnn molecules could be tissue markers of pathological situations in human (Fugmann et al., 2011; Jansen et al., 2009). A pantetheinase activity has also been detected in serum (Wittwer et al., 1989) but its molecular nature, origin and regulation were still unknown. Due to the poor sensitivity of available enzymatic assays (Dupre et al., 1984), we designed a novel substrate and ELISA to track mouse and human Vnn1 in tissues and body fluids. Preliminary results suggest that human and mouse Vnn1 share many similarities in expression pattern. However, unlike in mouse, human VNN1 transcripts have also been detected in PBL.

The purpose of this project was te generate a novel anti human VNN1 ELISA to quantify VNN1 expression in tissue and biological samples. More specifically, we propose to use VNN1 expression in feces as a marker of epithelial lesions in IBD.

### SUMMARY OF THE INVENTION:

The present invention provides vanin-1 (VNN1) as a biomarker for inflammatory bowel diseases. More particularly, VNN1 may be suitable for the diagnosis of inflammatory bowel disease (IBD), for the differential diagnosis between ulcerative colitis and Crohn's Disease, for monitoring the progression of an IBD, for detecting a bowel epithelial lesion in IBD, for monitoring treatments of IBD.

### DETAILED DESCRIPTION OF THE INVENTION:

### Definitions:

As used herein the term "inflammatory bowel disease" (IBD) describes a group of chronic inflammatory disorders of unknown causes involving the gastrointestinal tract. Patients with IBD can be divided into two major groups, those with ulcerative colitis (UC) and those with Crohn's disease (CD).

As used herein the term "vanin-1" or "VNN1" refers to has its general meaning in the art and refers to the protein having a pantetheinase activity (EC 3.5.1.92). An exemplary amino acid sequence is described in Genebank under the accession number CAA10568.

The tem biological sample has its general meaning in the art. Typically, the sample may be a blood sample obtained from the subject (e.g a serum sample) a faeces sample obtained from the patient, or a biopsy sample. Depending of the nature of the sample, the expression level of VNN1 may be determined according to the methods described in table A:

| Sample | Methods | Type of the value generated |
|---|---|---|
| Serum sample or faces | Methods comprising contacting the sample with a binding partner capable of selectively interactng with VNN1 protein (e.g. ELISA method) | Concentration value |
| Biopsy sample | Methods comprising contacting cells present in the sample with a binding partner capable of selectively interacting with VNN1protein ( e.g. immunochemistry method) | Number of VNN1 positive cells |
| | Methods comprising quantifying the gene expression level of VNN1 in the sample (e.g. a RT-PCR | Gene expression level |

***Methods for the early diagnosis of inflammatory bowel diseases:***

An object of the invention relates to a method for diagnosing an inflammatory bowel disease (IBD) in a subject comprising i) determining the expression level of vanin-1 (VNN1) expression in a biological sample obtained from the patient ii) comparing the level determined at step i) with a predetermined reference value wherein a difference between the expression level determined at step i) and the predetermined reference value indicates that the subject suffers from an inflammatory bowel disease.

In some embodiments, the method may further comprise the steps consisting of (iii) declaring a diagnosis of IBD or no IBD based on the difference observed in step (ii)," or "(iii) selecting a treatment option based on the difference observed in step (ii)," and optionally iv) further administering the selected treatment

Typically, the subject presents at least one clinical feature for which the diagnosis of an IBD is requested. Typically, the subject may suffer from chronic abdominal pain, diarrhea (often bloody), with or without extra-intestinal manifestations such as arthritis, uveitis, skin changes, etc.

Typically, the biological sample is a blood sample or a biopsy sample.

In one embodiment, the predetermined reference value is derived from the level of VNN1 in a control sample derived from one or more subjects who are substantially healthy as defined here above. Such subjects who are substantially healthy lack the clinical feature of IBD such as chronic abdominal pain and diarrhea (often bloody). Furthermore, IBD should be distinguished from IBS (Irritable Bowel Syndrome) where gut pain is not related to an ongoing inflammatory disorder. In another embodiment, such subjects are monitored and/or periodically retested for a diagnostically relevant period of time ("longitudinal studies") following such test to verify continued absence of IBD development. Such period of time may be one year, two years, two to five years, five years, five to ten years, ten years, or ten or more years from the initial testing date for determination of the predetermined reference value. Furthermore, retrospective measurement of VNN1 levels in properly banked historical subject samples may be used in establishing these predetermined reference values, thus shortening the study time required, presuming the subjects have been appropriately followed during the intervening period through the intended horizon of the product claim.

### Methods for the differential diagnosis between ulcerative colitis and Crohn's disease:

An object of the invention relates to a method for the differential diagnosis between ulcerative colitis and Crohn's disease in a subject comprising i) determining the expression level of vanin-1 (VNN1) expression in a biological sample obtained from the patient ii) comparing the level determined at step i) with at least one predetermined reference value wherein a difference between the expression level determined at step i) and the predetermined reference value indicates that the subject suffers from an ulcerative colitis or a Crohn's disease.

In some embodiments, the method may further comprise the steps consisting of (iii) declaring a diagnosis of ulcerative colitis or Crohn's disease based on the difference observed in step (ii)," or "(iii) selecting a treatment option based on the diagnosis that the subject has an ulcerative colitis or a Crohn's disease," and optionally iv) further administering the selected treatment.

Typically, the subject has been previously diagnosed with an IBD but the usual endoscopic, radiologic, and histopathologic criteria do not allow to the differential diagnosis between UC and CD.

Typically, the biological sample is a blood sample, a biopsy sample or faeces.

In one embodiment, the predetermined reference value is the level of VNN1 that permits the discrimination between UC and CD, with a high statistical significance value (e.g. low P value).

In one embodiment, the level determined at step i) is compared with a range of predetermined reference values. Typically, the range of the predetermined reference values is centered on the level of VNN1 for which the highest statistical significance value is found (e.g. generally the minimum P value which is found). For example, on a hypothetical scale of 1 to 10, if the ideal predetermined reference value (the value with the highest statistical significance) is 5, a suitable (exemplary) range may be from 4-6. Therefore, a subject may be assessed by comparing values obtained by determining the level of VNN1, where values greater than 5 indicate that the subject suffers from a CD or UC and values less than 5 indicate that the subject suffers from a CD or UC; or a patient may be assessed by comparing values obtained determining the level of VNN1 and comparing the values on a scale, where values above the range of 4-6 indicate that the subject suffers from a CD or UC.

### Methods for monitoring the progression of an IBD:

An object of the invention relates to a method for monitoring the progression of an IBD in a subject comprising i) determining the expression level of vanin-1 (VNN1) expression in a biological sample obtained from the patient ii) comparing the level determined at step i) with at least one predetermined reference value wherein a difference between the expression level determined at step i) and the predetermined reference value indicates that the IBD is an acute phase or a quiescent phase.

In some embodiments, the method may further comprise the steps consisting of (iii) declaring a diagnosis of an acute phase or a quiescent phase based on the difference observed in step (ii)," or "(iii) selecting a treatment option based on the diagnosis that the subject has an a quiescent phase or acute phase and optionally iv) further administering the selected treatment.

Typically, the subject has been previously diagnosed with an IBD.

Typically, the biological sample is a blood sample, a biopsy sample or faeces.

In one embodiment, the predetermined reference value is the level of VNN1 that permits the discrimination between a quiescent phase and an acute phase, with a high statistical significance value (e.g. low P value).

In one embodiment, the level determined at step i) is compared with a range of predetermined reference values. Typically, the range of the predetermined reference values is centered on the level of VNN1 for which the highest statistical significance value is found (e.g. generally the minimum P value which is found). For example, on a hypothetical scale of 1 to 10, if the ideal predetermined reference value (the value with the highest statistical significance) is 5, a suitable (exemplary) range may be from 4-6. Therefore, a subject may be assessed by comparing values obtained by determining the level of VNN1, where values greater than 5 indicate that the IBD is an acute phase and values less than 5 indicate that the IBD is a quiescent phase; or a patient may be assessed by comparing values obtained determining the level of VNN1 and comparing the values on a scale, where values above the range of 4-6 indicate that the IBD is an acute phase and values below the range of 4-6 indicate that IBD is a quiescent phase.

### Methods for detecting a bowel epithelial lesion in IBD:

An object of the invention relates to a method for detecting a bowel epithelial lesion in a subject suffering from an IBD comprising i) determining the expression level of vanin-1 (VNN1) expression in a biological sample obtained from the patient ii) comparing the level determined at step i) with at least one predetermined reference value wherein a difference between the expression level determined at step i) and the predetermined reference value indicates a bowel epithelial lesion occurs.

In some embodiments, the method may further comprise the steps consisting of (iii) declaring a bowel epithelial lesion based on the difference observed in step (ii)," or "(iii) selecting a treatment option based on the diagnosis that the subject has a bowel epithelial lesion or not and optionally iv) further administering the selected treatment.

Typically, the biological sample is a blood sample or a biopsy sample.

In one embodiment, the predetermined reference value is derived from the level of VNN1 in a control sample derived from one or more subjects for whom a bowel epithelial lesion does not occur.

### Methods for monitoring treatments of IBD:

An object of the invention relates to a method for monitoring the effectiveness of a treatment of an inflammatory bowel disease in a subject comprising i) determining the expression level of vanin-1 (VNN1) expression in a biological sample obtained from the patient before the treatment ii) determining the expression level of vanin-1 (VNN1) expression in a biological sample obtained from the patient after the treatment, iii) comparing the expression level of vanin-1 (VNN1) determined at step i) with the expression level of vanin-1 (VNN1) determined at step ii) and iv) and concluding that the treatment is efficient when the expression level determined at step ii) is lower than the expression level determined at step i) or concluding that the treatment is inefficient when the expression level determined at step ii) is about the same or higher than the expression level determined at step i).

Typically, the biological sample is a blood sample, a biopsy sample or a faeces sample.

Current therapies comprise aminosalicylates, drugs that contain 5-aminosalicyclic acid (5-ASA), which help to control inflammation. Sulfasalazine is mainly useful in colonic disease because the active compound, 5-aminosalicylic acid (5 -ASA), is released in the large bowel by bacterial degradation of the parent compound. Products such as mesalamine that release 5-ASA in the distal small bowel secondary to pH changes are more useful in patients with small intestinal Crohn disease. Other 5-ASA agents, such as olsalazine, mesalamine, and balsalazide, have a different carrier, fewer side effects, and may be used by people who cannot take sulfasalazine. Immunomodulators such as azathioprine and 6-mercapto-purine reduce inflammation by affecting the immune system. Since TNF is a key inflammatory cytokine and mediator of intestinal inflammation, compounds directed against this cytokine such as infliximab are promising in IBD. In certain cases, immunosuppressive agents: cyclosporine A, tacrolimus, mycophenolate mofetil may be effective in treating IBD. Glucocorticoids such as prednisone, methylprednisone, and hydrocortisone also reduce inflammation. They can be given orally, intravenously, through an enema, or in a suppository, depending on the location of the inflammation. These drugs can cause side effects such as gastrointestinal ulceration, weight gain, acne, facial hair, hypertension, diabetes, mood swings, bone mass loss, and an increased risk of infection. For this reason, they are not recommended for long-term use, although they are considered very affective when prescribed for short-term use.

### Methods for determining the level of a protein in a biological sample

According to the invention, determining the expression level of a protein in a biological sample (e.g. a blood sample or a faeces sample) can be performed by a variety of techniques. Typically, the methods may comprise contacting the sample with a binding partner capable of selectively interacting with the protein of interest in the sample. In some aspects, the binding partners are antibodies, such as, for example, monoclonal antibodies or even aptamers as above described.

The aforementioned assays generally involve the binding of the partner (ie. antibody or aptamer) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

The level of the protein of interest may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation.

An exemplary biochemical test for identifying specific proteins employs a standardized test format, such as ELISA test, although the information provided herein may apply to the development of other biochemical or diagnostic tests and is not limited to the development of an ELISA test (see, e.g., Molecular Immunology: A Textbook, edited by Atassi et al. Marcel Dekker Inc., New York and Basel 1984, for a description of ELISA tests). It is understood that commercial assay enzyme-linked immunosorbant assay (ELISA) kits for various plasma constituents are available. Therefore ELISA method can be used, wherein the wells of a microtiter plate are coated with a set of antibodies which recognize the protein of interest. A sample containing or suspected of containing the protein of interest is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art.

Measuring the level of the protein of interest (with or without immunoassay-based methods) may also include separation of the compounds: centrifugation based on the compound's molecular weight; electrophoresis based on mass and charge; HPLC based on hydrophobicity; size exclusion chromatography based on size; and solid-phase affinity based on the compound's affinity for the particular solid-phase that is used. Once separated, said one or two biomarkers proteins may be identified based on the known "separation profile" e. g., retention time, for that compound and measured using standard techniques.

Alternatively, the separated compounds may be detected and measured by, for example, a mass spectrometer.

Typically, levels of the immunoreactive protein of interest in a sample may be measured by an immunometric assay on the basis of a double-antibody "sandwich" technique, with two monoclonal antibodies specific for the protein of interest (Cayman Chemical Company, Ann Arbor, Michigan). Preferably, the antibodies have no cross-reactivity with the other types of neighbouring proteins (e.g. VNN2 or VNN3). According to said embodiment, said means for measuring the protein of interest level are for example i) the protein of interest in a buffer, ii) two monoclonal antibodies that interact specifically with the protein of interest, iii) an enzyme-conjugated antibody specific for the protein of interest and a reference value of the protein of interest.

### Methods for determining the expression level of a gene:

Determination of the expression level of a gene can be performed by a variety of techniques. Generally, the expression level as determined is a relative expression level. In a preferred embodiment, the expression level may be determined by determining the quantity of mRNA.

Methods for determining the quantity of mRNA are well known in the art. For example the nucleic acid contained in the samples (e.g., cell or tissue prepared from the subject) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210)

### Methods for determining the level of positive cells for a biomarker:

Determining the level of positive cells for a biomarker may be determined by any well known method in the art. Typically, such methods comprise contacting the biopsy sample (e.g. tissue sample) with a binding partner capable of selectively interacting with the biomarker (i.e VNN1). The binding partner may be polyclonal antibody or monoclonal antibody, an antibody fragment, synthetic antibodies, or other protein-specific agents such as nucleic acid or peptide aptamers. However, monoclonal antibodies specific for the biomarker are preferred for this purpose due to specificity and availability. The preferred method according to the present invention is immunohistochemistry. The use of antibodies to identify proteins of interest in the cells of a tissue, referred to as immunohistochemistry (IHC), is well established. See for exemple "Principles and practice of immunoassays" 1991, C.P. Price and D.J. Neoman (eds) Stockton Press, N.Y. For the detection of the antibody that makes the presence of the protein of interest detectable by microscopy or an automated analysis system, biomarker antibodies may be tagged directly with detectable labels such as enzymes, chromogens or fluorescent probes or indirectly detected with a secondary antibody conjugated with detectable labels. The preferred staining method according to the present invention uses a secondary antibody coupled to an amplification system (to intensify staining signal) and enzymatic molecules. Such coupled secondary antibodies are commercially available, e.g. from Dako, EnVision system. Counterstaining may be used, e.g. H&E, DAPI, Hoechst. Other staining methods may be accomplished using any suitable method or system as would be apparent to one of skill in the art, including automated, semi-automated or manual systems.

The invention will be further illustrated by the following figures and examples. However, these examples and figures should not be interpreted in any way as limiting the scope of the present invention.

### FIGURES:

**Figure 1** shows Vnn1 expression in mouse gut by qRT-PCR.
**Figure 2** shows the quantification by ELISA of Vnn1 in faeces following DSS colitis induction
**Figure 3** shows the expression analysis of human VNN1 by immunohistochemistry in gut from control or IBD patients
**Figure 4** shows the expression analysis of human VNN1 by qRT-PCR in gut from control or IBD patients.
**Figure 5** shows the VNN1 ELISA calibration using recombinant VNN1
**Figure 6** shows the correlation between VNN1 ELISA and enzymatic activity detected using the pAMC substrate

### EXAMPLE:

### Material & Methods

The VNN1 recombinant molecule was obtained from Sino Biological Inc.

### Vnn1 cDNA

A tagged version of VNN1 cDNA was transfected into murine cell lines and expression was verified by quantifying enzymatic activity and surface expression

### Vanin transfectants

Both mouse and human transfectants were produced in the mouse MTE1D epithelial or NIH3T3 fibroblastic cell lines.

### Immunization procedure

Two protocols were used for the production of rat ou mouse anti-human VNN1. The first protocol consisted in administering VNN1 MTE1D transfectants ip in rats (3 injections of 107 cells every 15 days), followed by a boost 3, 2 and 1 day before the fusion. Rat splenocytes were fused with the X63 myeloma and hybridomas selected for the production of anti-VNN1 antibodies by flow cytometry on the NIH3T3 transfected with control versus human VNN1 cDNA. The 34G7 hybridoma was selected in this first fusion. The second protocol was based on the injection of 10 µg of recombinant VNN1 molecule in CFA ip in Vnn1-deficient mice. After two boosts ip in IFA, the recombinant protein was administered iv 3 days before the fusion. Mouse splenocytes were harvested and fused with the X63 mouse myeloma. Hybridomas secreting anti-VNN1 mAbs were screened by ELISA using another form of human VNN1 bearing a Flag tag allowing its capture on Rabbit anti-Flag antibody-coated plates. Positive wells were then retested on VNN1 cell transfectants. The 6E3.5 mouse hybridoma was selected in this second fusion.

### Flow cytometry

Analysis of hybridoma supernatants was performed by flow cytometry on VNN1 cell transfectants. Samples were processed on a LSR flow cytometer (Becton Dickinson) using fluorochrome coupled secondary reagents.

### Results

### Vnn1 expression in mouse gut is increased during DSS colitis

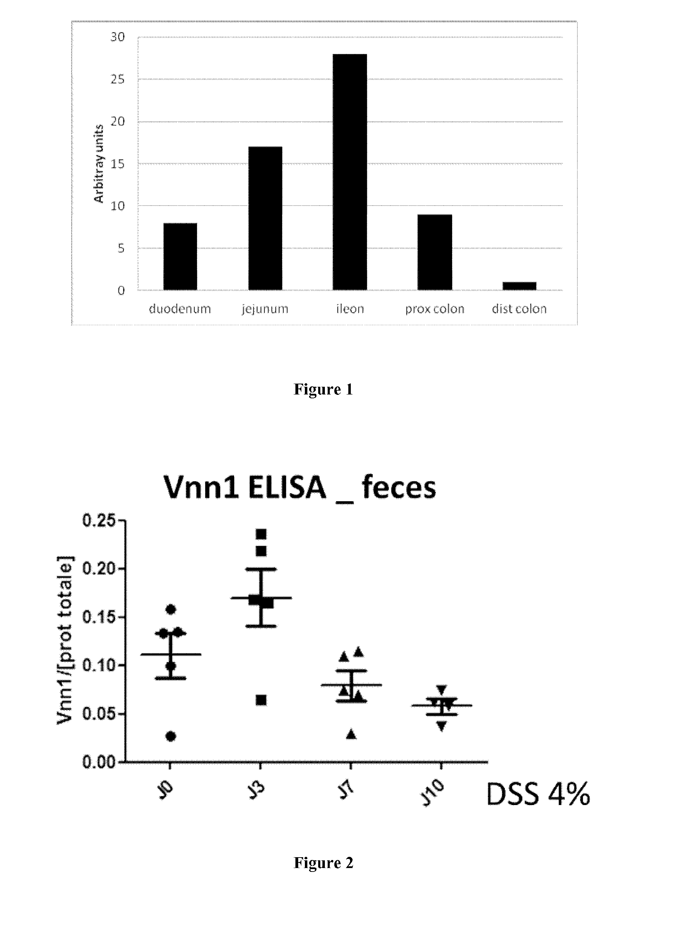

We first verified that Vnn1 expression is expressed at the brush border of ileal mucosa by immunohistochemistry (data not shown). We then performed a systematic analysis of Vnn1 expression by qRT-PCR on various sections of mouse gut. As shown in Figure 1, expression is maximal in distal ileon and much weaker on normal colon. Interestingly, expression is enhanced in colon following administration of 4%DSS for 7 days (data not shown).

### Vnn1 is detected in the feces during the course of the colitis

Since Vnn1 is mostly epithelial in mouse, we reasoned that during the development of the colitis, the destruction of the mucosa might be accompanied by the excretion of Vnn1 in the feces. As seen in Figure 2, we could easily detect the Vnn1 protein by ELISA 3 days after the beginning of the DSS before the development of clinical symptoms and weight loss. Vnn1 excretion progressively returned to undetectable levels at the end of DSS administration. Therefore Vnn1 excretion is an early marker of ongoing colitis in mouse and might represent an early sign of mucosal degradation.

### VNN1 expression on human colon

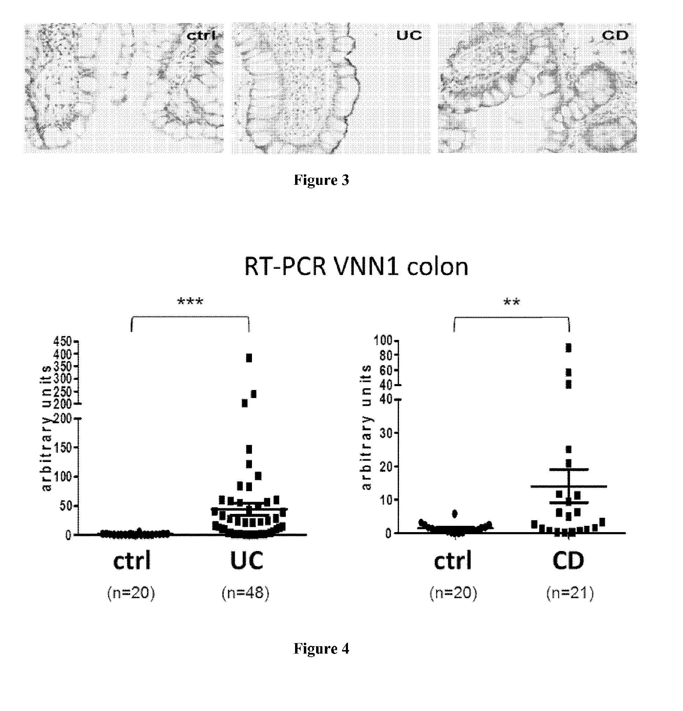

We thus decided to validate these results in human. We first produced an anti-human VNN1 mAb able to detect VNN1 on formal-fixed human biopsies from control or IBD patients. VNN1 is poorly detectable in the colon of control individuals but is highly expressed on the at the brush border of mucosal epithelial cells of UC patients (Figure 3). Expression in CD patients is more heterogeneous with areas of high expression intermingled with subnormal areas. Interestingly, VNN1 expression could only be detected. No staining was detected in the submucosal tissue even in areas with high infiltration by immunocytes. This result suggests that if excreted, VNN1 should be a valid marker of mucosal cells as in mouse. This is in agreement with the fact that erosive UC lesions are largely distributed on the colonic mucosa whereas CD mucosal alterations develop around dispersed granulomatous lesions surrounded by areas of healthy mucosa. This expression pattern was then checked by qRT-PCR on biopsies from IBD patients. As shown in Figure 4, most patients with UC show significant increase of VNN1 during flares but also elevated basal levels during remissions. VNN1 expression is also higher in CD patients but the range of overexpression is more limited, probably in part due to the heterogeneity of VNN1 overexpression in the tissue. Nevertheless these results suggest that VNN1 could be a marker of inflamed gut mucosa during IBD, in agreement with mouse data.

### VNN1 ELISA

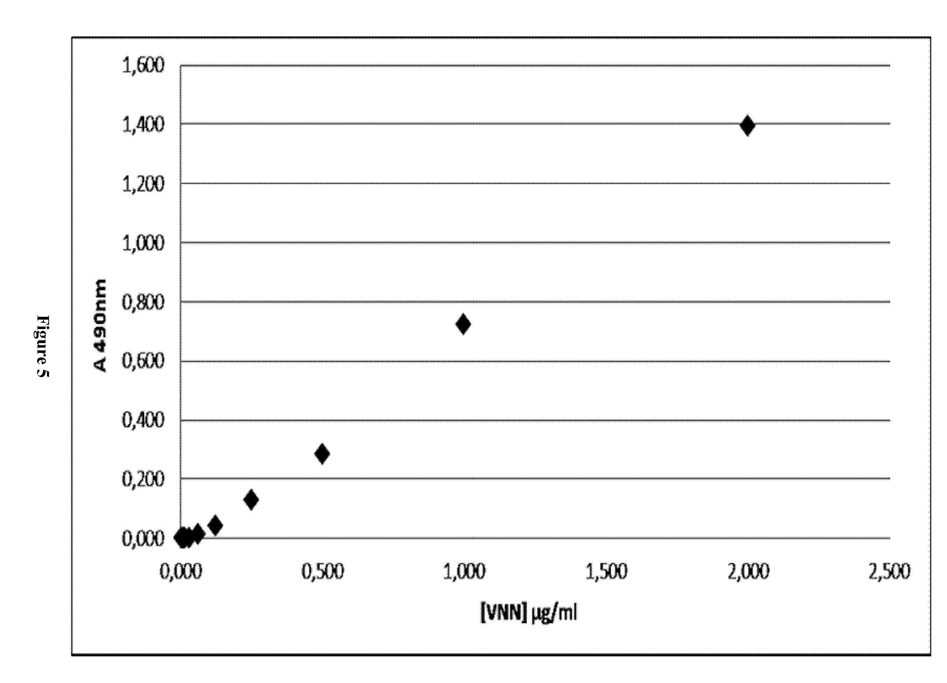

Using the strategy described in the MM section, we were able to generate anti-VNN1 mAbs against different epitopes of the VNN1 molecule. Indeed, using recombinant human VNN1 injection into Vnn1-deficient host mice, we increased the immunogenicity spectrum between mouse and human isoforms. A large panel of anti-VNN1 mAbs was produced and tested for their ability to bind VNN1 transfected cells in the presence of the 34G7 mAb. We selected by flow cytometry on VNN1 transfectants, antibodies that did not compete for related epitopes. Some of them were purified and tested for the design of the ELISA using the biotinylated 34G7 mAb as a detecting mAb. As shown in Figure 5, when coated on 96-well plates, the 6E3.5 mAb can capture a recombinant VNN1 protein. The sensitivity of the assay was high enough to detect at least 60 ng/ml VNN1 and was linear up to 3 µg/ml. We further validated the specificity of this ELISA on cells transfected with control of VNN1 cDNA (not shown).

### Validation on human serum

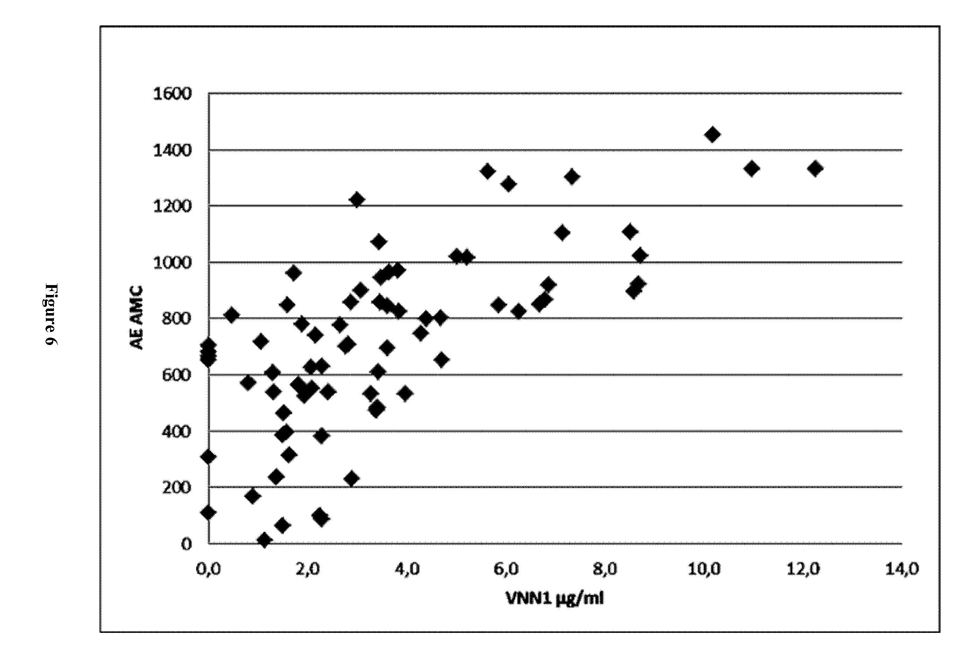

Since recombinant or transfected VNN1 molecules might not carry the same post-traductional modifications as native proteins in vivo, we validated the ELISA on samples from control human individuals. It has been shown that pantetheinase could be detected in serum and our unpublished results demonstrated the presence of mouse Vnn1 in the serum of mice. As shown in Figure 6, human VNN1 is easily detected in the serum of patients at a level close to 12µg /ml. We then compared the level of the VNN1 protein to that of the enzymatic activity using the pAMC substrate previously shown to be relatively specific for the VNN1 isoform. As shown if Figure 5, there is a remarkable correlation between the level of VNN1 protein and enzymatic activity.

### REFERENCES:

Throughout this application, various references describe the state of the art to which this invention pertains. The disclosures of these references are hereby incorporated by reference into the present disclosure.

## Claims

1. A method for diagnosing an inflammatory bowel disease (IBD) in a subject comprising i) determining the expression level of vanin-1 (VNN1) expression in a biological sample obtained from the patient ii) comparing the level determined at step i) with a predetermined reference value wherein a difference between the expression level determined at step i) and the predetermined reference value indicates that the subject suffers from an inflammatory bowel disease.

2. A method for the differential diagnosis between ulcerative colitis and Crohn's disease in a subject comprising i) determining the expression level of vanin-1 (VNN1) expression in a biological sample obtained from the patient ii) comparing the level determined at step i) with at least one predetermined reference value wherein a difference between the expression level determined at step i) and the predetermined reference value indicates that the subject suffers from an ulcerative colitis or a Crohn's disease.

3. A method for monitoring the progression of an IBD in a subject comprising i) determining the expression level of vanin-1 (VNN1) expression in a biological sample obtained from the patient ii) comparing the level determined at step i) with at least one predetermined reference value wherein a difference between the expression level determined at step i) and the predetermined reference value indicates that the IBD is an acute phase or a quiescent phase.

4. A method for detecting a bowel epithelial lesion in a subject suffering from an IBD comprising i) determining the expression level of vanin-1 (VNN1) expression in a biological sample obtained from the patient ii) comparing the level determined at step i) with at least one predetermined reference value wherein a difference between the expression level determined at step i) and the predetermined reference value indicates a bowel epithelial bowel disease occurs.

5. A method for monitoring the effectiveness of a treatment of an inflammatory bowel disease in a subject comprising i) determining the expression level of vanin-1 (VNN1) expression in a biological sample obtained from the patient before the treatment ii) determining the expression level of vanin-1 (VNN1) expression in a biological sample obtained from the patient after the treatment, iii) comparing the expression level of vanin-1 (VNN1) determined at step i) with the expression level of vanin-1 (VNN1) determined at step ii) and iv) and concluding that the treatment is efficient when the expression level determined at step ii) is lower than the expression level determined at step i) or concluding that the treatment is inefficient when the expression level determined at step ii) is about the same or higher than the expression level determined at step i).
